# FASCICULE DE BREVET EUROPEEN

(11) **EP 1 043 018 B1**
(45) Date de publication et mention de la délivrance du brevet: **14.06.2006**
(21) Numéro de dépôt: 00400490.9
(22) Date de dépôt: 23.02.2000
(51) Int. Cl.: A61K 8/02, A61Q 19/00, A61K 9/70

(54) **Patch à effet de champ magnétique**
Pflaster mit magnetischer Wirkung
Magnetic field effect patch

(30) Priorité: 31.03.1999 FR 9904043
(43) Date de publication de la demande: 11.10.2000
(73) Titulaire: L'OREAL, 75008 Paris (FR)
(72) Inventeur: Gueret, Jean-Louis H., 75016 Paris (FR)
(74) Mandataire: Schmit, Charlotte

(56) Documents cités:
- WO-A-96/14822
- WO-A-96/37283
- DE-A- 3 613 280
- DE-A- 3 619 987
- DE-A- 4 325 071
- DE-A- 19 715 477
- DE-A- 19 715 478
- DE-U- 8 610 769
- FR-A- 2 776 518
- PATENT ABSTRACTS OF JAPAN vol. 016, no. 256 (C-0969) 31 Juillet 1992 & JP 04 108 710 A (YOKO SHIGA) 09 Avril 1992

## Description

La présente invention concerne un patch destiné à être appliqué temporairement sur la peau pour exercer sur celle-ci une action cosmétique et/ou un traitement pharmaceutique.

On connaît des patchs, notamment du document WO-96/14822, comportant une feuille de support revêtue sur une face d'une couche formant réservoir appelée matrice, contenant une ou plusieurs substances actives destinées à diffuser dans la peau et/ou à agir sur celle-ci.

Dans le cas d'un traitement de la peau, à partir d'actifs destinés à avoir une action notamment en profondeur, l'efficacité du traitement peut tenir, outre au choix du (ou des) bon(s) actif(s), à l'aptitude de ces actifs à pénétrer dans la peau. Une telle aptitude tient à la fois aux propriétés intrinsèques de l'actif, mais également à la peau elle même. A cet effet, il est possible d'agir sur la peau pour en favoriser sa réceptivité aux actifs.

Dans le passé, il a été mis en évidence qu'un champ magnétique appliqué sur la peau pouvait avoir une action bénéfique, notamment sur la micro circulation ainsi que sur différents aspects du métabolisme. Il est également bien connu que l'amélioration de la micro circulation peut jouer un rôle notamment amincissant, ou encore, favoriser la pénétration d'actifs, notamment cosmétiques. Un des problèmes qui, à ce jour n'a pas été résolu, tient à la mise en oeuvre pratique d'un tel champ magnétique, en combinaison avec l'application du ou des actifs, sans générer pour l'utilisatrice de manipulations coûteuses en temps et fastidieuses. Dans cette optique, il a été essayé, sans grand succès, d'incorporer des particules magnétiques dans des crèmes, pâtes, une huiles ou lotions.

Ainsi, le modèle d'utilité Allemand DE 86 10 769.0 suggère d'incorporer dans une crème destinée à former un masque, de beauté ou de soin, des petits aimants, ayant un moment magnétique avec au moins un pôle nord et un pôle sud. La crème est minérale, notamment à base d'argile, de vaseline ou de plâtre. La taille des aimants peut aller de 0,5 mm à 30 mm, et de préférence, de 2 mm à 10 mm.

La demande DE-A-36 19 987, fait état d'un problème majeur dont souffre la structure décrite dans le document DE 86 10 769.0, mentionné ci-avant. Ce problème tient au fait que, les aimants, en raison de leur aimantation permanente, ont tendance à s'attirer, ou à se repousser, et ainsi, former des paquets. Dans la préparation qui en résulte, les aimants ne sont pas dispersés de manière homogène. Les résultats à l'application ne sont pas satisfaisants. Pour résoudre ce problème, le document DE-A-36 19 987 propose de coller les aimants sur un support, et d'enduire ensuite le support de ladite préparation cosmétique. L'utilisateur applique ainsi la préparation telle que déposée sur un support. Une telle solution souffre principalement du fait que la matrice minérale, notamment quand elle est réalisée à base de plâtre ou d'argile, va sécher et durcir ou bout d'un certain temps d'application, procurant une sensation d'inconfort relativement importante. La rigidité de l'ensemble est renforcée par la présence des aimants, de taille relativement importante, collés sur le support. En outre, la répartition des aimants dans la préparation n'est pas homogène, ce qui peut être pénalisant en termes d'efficacité.

De certains documents, en particulier du document DE -A-197 15 478, il est connu d'introduire des particules magnétiques, notamment des ferrites, dans une composition cosmétique ou dermatologique. Les particules ne sont pas magnétisées de façon permanente.

Aussi, est-ce un des objets de l'invention que de pouvoir réaliser une structure, en particulier sous forme d'un patch, cosmétique ou de soin, formée d'une matrice dans laquelle sont dispersées des particules magnétiques, orientées de façon permanente.

La demanderesse a découvert de manière surprenante qu'un tel effet de champ magnétique pouvait être obtenu, de manière simple et efficace, en incorporant le (ou les) actifs à utiliser dans la matrice polymérique d'un patch, de préférence adhésive, et en dispersant dans la matrice une quantité de particules magnétiques, que l'on oriente de façon permanente, après figeage de la matrice, en faisant passer le patch dans un champ magnétique. On met ainsi la peau en engagement, et ce de manière simultanée, avec un ou plusieurs actifs, et avec des particules magnétiques, orientées de manière permanente, de manière notamment à favoriser la pénétration des actifs dans la peau.

Ainsi, la présente invention propose un patch selon la revendication 1 . La matrice polymérique est de préférence anhydre ou hydrophobe. De préférence, les particules magnétiques sont en dispersion de façon homogène dans la matrice.

La matrice, une fois polymérisée au moins partiellement, notamment par réticulation, polyaddition ou polycondensation, forme une structure figée dans laquelle sont immobilisées les particules magnétiques. Dans cette configuration, les particules de tout ou partie du patch, peuvent être soumises à un champ magnétique, de manière à être magnétisées de façon permanente. Ainsi figées dans la matrice polymérique, les particules restent dispersées de manière homogène dans la matrice et conservent leur orientation magnétique. A l'application sur la peau, les effets sont uniformes sur toute la surface magnétisée du patch.

Ainsi, lors de l'application, les lignes de champ magnétiques, générées par les particules magnétiques orientées de façon permanente, ont une action bénéfique notamment sur l'oxygénation de la peau, et sur la micro circulation, contribuant ainsi à favoriser la pénétration des actifs dans la peau et à en renforcer l'efficacité. Les temps de pose des patchs selon l'invention peuvent être réduits de façon sensible. Les concentrations de certains actifs, jugés agressifs pour la peau, peuvent être réduites de façon notable. En outre, les particules magnétiques peuvent elle mêmes jouer le rôle de substance active, dans la mesure où le champ magnétique qu'elles génèrent, peut avoir, en agissant sur la micro circulation, un effet, notamment amincissant.

Lesdites particules magnétiques peuvent être notamment des particules de ferrite, à base notamment de zinc et de manganèse. Alternativement, on utilise des particules commercialisées sous la dénomination commerciale MagaBeads® , par la société CORTEX BIOCHEM.

Le pourcentage en volume desdites particules magnétiques à l'intérieur de la matrice polymérique peut aller de 0,1 à 80 %, et de préférence, de 1 % à 30 %, et de préférence encore, de 2 % à 15 %.

Lesdites particules peuvent être enrobées, notamment de polyuréthanne, d'époxy, de polyester, de polyamide, ou de cyanoacrylate. Un tel enrobage participe à la protection des particules magnétiques lorsqu'elles ont mises en présence d'eau, notamment sous l'effet de la transpiration de la peau.

Les particules magnétiques peuvent être orientées de sorte que, sur au moins une portion de la surface du patch, les lignes de champ magnétique générées s'étendent depuis une première face du patch jusqu'à une seconde face, opposée à la première. Alternativement, les particules magnétiques sont orientées de sorte que, sur au moins une portion de la surface du patch, les lignes de champ magnétique générées s'étendent depuis un premier bord du patch jusqu'à un second bord, opposé au premier. D'autres arrangements ou combinaisons encore peuvent être obtenus, notamment en multipliant les passages dans des champs magnétiques identiques ou différents, ou en jouant sur les caractéristiques du banc d'aimantation utilisé.

La (ou les) substances active(s) peuvent être choisies parmi les agents amincissants, nettoyants, anti-oxydants, anti-radicaux libres, hydratants, dépigmentants, liporégulateurs, anti-acnéiques, anti-séborrhéiques, anti-vieillissement, adoucissants, anti-rides, kératolitiques, anti-inflammatoires, rafraîchissants, cicatrisants, protecteurs vasculaires, anti-bactériens, antifongiques, anti-perspirants, déodorants, conditionneurs de la peau, insensibilisants, immunomodulateurs et nourrissants, absorbeurs d'humidité (coton, polyacrylate), de sébum (Orgasol).

La matrice polymérique peut comporter au moins une substance active hydrosoluble choisie parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

En outre, la matrice polymérique peut comporter au moins une substance active liposoluble choisie parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

De tels actifs peuvent être incorporés à l'état solubilisé dans des huiles, utilisées seules ou en combinaison, parmi lesquelles on peut citer : les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, de pistache, d'abricot, d'amandes ou d'avocat; les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin ; l'huile de germes de blé, de calophyllum, de sésame, de coriandre, de carthame, l'huile de passiflore, l'huile de rosa mosqueta, de macadamia, de pépins de fruits (raisin, cassis, orange, kiwi), de colza, de coprah, d'arachide, d'onagre, de palme, de ricin, de lin, de jojoba, de chia, d'olive ou de germes de céréales comme l'huile de germes de blé, l'huile de son de riz, l'huile de karité ; des acétylglycérides; des octanoates, décanoates ou ricinoléates d'alcools ou de polyalcools ; des triglycérides d'acides gras ; des glycérides ; l'huile de paraffine, de vaseline, le perhydrosqualène ; des alcools gras (alcool stéarylique, alcool cétylique) et des acides gras (acide stéarique) et leurs esters ; les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA).

On peut encore citer les huiles hydrocarbonées partiellement fluorées ou les huiles perfluorées, et notamment les perfluoropolyéthers et les perfluoroalcanes.

La phase huileuse, c'est-à-dire les gouttelettes d'huile dispersées dans la matrice polymérique, peut être présente dans une proportion pouvant aller de 0,1% à 30% en poids par rapport au poids total de la composition. De façon préférentielle, ce pourcentage est compris entre 5 et 25 %.

Alternativement, le composé actif liposoluble est incorporé dans une couche de polymère hydrophobe, sous forme de poudre ou de granulés.

Avantageusement, la matrice polymérique peut contenir au moins une substance active ayant un effet nettoyant sur la peau, ledit patch comportant une couche colorée de manière à pouvoir, de visu, quantifier et/ou qualifier les impuretés prélevées de la peau par ladite surface adhésive.

La présence de la couche colorée, rend détectables de visu, et permet de quantifier (en nombre et en taille) et/ou qualifier les impuretés sur la surface colorée du patch. La quantité d'impuretés prélevées est représentative de la fréquence souhaitable d'application dudit patch. Ainsi, la présence d'une quantité importante de tels résidus indique à l'utilisatrice qu'elle doit appliquer le patch sur la base d'une fréquence relativement élevée (par exemple, tous les jours). Une quantité plus faible de telles impuretés indique que la fréquence d'utilisation doit être plus faible (par exemple, une fois par semaine). En outre, le type d'impuretés prélevées permet à l'utilisatrice de choisir au mieux le type de traitement nécessaire à sa peau.

Les pigments colorés peuvent être constitués notamment de pigments du type de ceux utilisés dans le domaine de l'alimentaire ou de la cosmétique, en particulier pour les rouges à lèvres ou les vernis à ongles. A titre d'exemple, on peut citer, seuls ou en combinaison, des pigments synthétiques, ou des pigments minéraux, notamment des pigments d'oxyde de zirconium ou de cérium, ainsi que les oxydes de fer ou de chrome, et le bleu ferrique. On peut utiliser des pigments organiques, notamment le noir de carbone, les laques de baryum, strontium, calcium (DC Red N°7), aluminium. A titre d'exemple plus spécifique, on utilise un pigment portant la référence DC violet 2 K7014, commercialisé par KOHNSTAMM® .

La matrice polymérique peut contenir au moins une substance active choisie parmi les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits et les bêta-hydroxyacides comme l'acide salicylique et ses dérivés notamment alcoylés comme, l'acide n-octanoyl-5-salicylique ; de la poudre de Kaolin, des particules de polyamide (ORGASOL® ), des cires, du miel, de la terre de Sienne, des tanins ou des sels minéraux.

La matrice polymérique peut être constituée d'une matrice auto-adhésive (sur peau sèche et/ou sur peau mouillée) à base notamment d'adhésif polyacrylique, polyvinylique, ou d'une matrice hydrophobe à base d'un polymère de silicone ou de polyuréthanne, dont la réticulation est de préférence partielle de manière à lui conférer une adhérence sans requérir de couche adhésive supplémentaire. On peut utiliser également une matrice adhésive en latex, butyle, ou tout autre adhésif élastomère.

A titre d'exemple avantageux, on utilise pour réaliser la matrice polymérique, un composé polyacrylique, en solution dans un solvant, notamment de l'acétate d'éthyle ou de l'alcool. L'adhérence du patch peut être comprise entre 50 g/cm² et 800 g/cm² (force exercée perpendiculairement au plan de la surface adhésive, nécessaire à son décollement de la peau), et de préférence entre 100 et 700 g/cm², et de préférence encore entre 300 et 600 g/cm². La surface de la matrice destinée à venir en contact avec la peau peut être lisse ou présenter des aspérités ou reliefs.

Avantageusement, la matrice auto-adhésive peut contenir des particules d'au moins un agent hydroabsorbant en dispersion de façon homogène dans ladite matrice. En effet, au contact notamment de l'humidité de la peau, les particules de l'agent hydroabsorbant captent de l'eau, favorisant ainsi la solubilisation du composé actif solide, hydrosoluble. En quelque sorte, par cette solubilisation "in situ" de l'actif hydrosoluble, sa biodisponibilité est quasi instantanée, et toute interaction éventuelle avec les autres composés présents dans la couche polymérique est minimisée. L'humidité de la peau peut jouer le rôle de solubilisant de l'actif hydrosoluble d'autant plus, que la couche support du patch et/ou la matrice peuvent créer des conditions occlusives.

Parmi les agents hydroabsorbants pouvant être présents dans la matrice polymérique hydrophobe à l'état dispersé, on peut citer, de préférence, les polyacrylates réticulés superabsorbants à fort taux de gonflement dans l'eau, tels que ceux commercialisés par la Société NORSOLOR sous la dénomination *Aquakeep*® ; l'alcool polyvinylique ; les polymères carboxyvinyliques tels que ceux commercialisés par la Société GOODRICH sous les dénominations de "Carbopol"® ; les dérivés semi-synthétiques de la cellulose tels que la carboxyméthylcellulose ; les substances naturelles telles que les amidons, les gommes naturelles (gomme de guar, gomme arabique, gomme adragante), la caséine, les phytocolloïdes (carragénates, alginates, agar-agar), les fibres de coton et la gélatine.

On préfère tout particulièrement utiliser les polyacrylates réticulés superabsorbants dont la présence à l'état dispersé dans une matrice polymérique hydrophobe permet de pomper la transpiration par exemple, et favorise, après hydratation, un meilleur contact avec les particules des autres actifs présents le cas échéant dans la matrice.

L'agent hydroabsorbant tel que défini ci-dessus est présent, de préférence, dans une proportion allant d'environ 0,2 % à environ 20 % en poids, et plus particulièrement allant de 0,5 % à 10 % par rapport au poids total de la couche de polymère.

Alternativement, la matrice est formée d'au moins un polymère hydrosoluble, apte, après hydratation, à former un gel qui, en séchant, adhère à la peau, A titre d'exemple, on peut citer les PVP ou PVA. Avec une telle configuration, le patch peut être appliqué sec sur peau mouillée, ou mouillé sur peau sèche. De manière générale, la première solution est préférée.

Alternativement encore, la matrice polymérique est formée d'un gel à forte teneur en eau, de type hydrogel, notamment à base d'au moins un hydrocolloïde, et présentant une adhésion de contact, similaire à celle résultant d'un effet ventouse. De tels gels peuvent être des gels réversibles ou irréversibles.

Le ou les hydrocolloïdes utilisés, peuvent être par exemple choisis dans le groupe formé par :
- la gomme de gellane ;
- la cellulose ou ses dérivés comme la carboxyméthylcellulose, l'hydroxypropylcellulose, la méthylcellulose, l'hydroxypropylméthylcellulose ou l'hydroxyéthylcellulose ainsi que les celluloses modifiées notamment par greffage de groupement alkyle ;
- les extraits d'algue tels que l'agar-agar, les carraghénanes, les alginates ;
- les extraits de graines tels que la gomme de caroube, la gomme de guar, les gommes de guar modifiées notamment par greffage de groupement alkyle ;
- les exsudats de plantes tels que la gomme arabique, la gomme karaya, la gomme adragante, la gomme de gatty ;
- les exsudats de micro-organismes tels que la gomme de xanthane ;
- les extraits de fruits tels que les pectines ;
- les agents gélifiants d'origine animale tels que la gélatine, les caséïnates ;
- les polymères synthétiques gélifiants hydrosolubles tels que les acides polyacryliques réticulés, éventuellement par une chaîne alkyle, tels que les "Carbopol" ou " Pemulen" de la Société GOODRICH ;
- les dérivés du silicium tels que les hectorites synthétiques comme les produits "Laponite RD et RDS" vendus par la société WAVERLY, les silicates d'aluminium et de magnésium comme le produit "Veegum" vendu par la société VANDERBILT,
- les produits du type POLYCARE® , commercialisé par la société Rhone-Poulenc,
ou un mélange de ces composés.

De préférence, le support, sur lequel la matrice polymérique est déposée, peut être constitué de tout matériau approprié imperméable aux composés actifs contenus dans la matrice adjacente. La couche support a non seulement pour fonction de supporter la matrice mais également de servir de revêtement protecteur de celle-ci. Elle peut être de même dimension que la matrice ou de dimension plus grande de telle sorte qu'elle s'étende au-delà la périphérie de la matrice.

Le support peut être un support occlusif. A titre d'exemple, le support est constitué d'un matériau thermoplastique, choisi parmi les polyéthylènes haute et basse densité, les polypropylènes, les polychlorures de vinyle, les copolymères d'éthylène et d'acétate de vinyle, les polyesters et les polyuréthannes, ou d'un complexe de tels matériaux. Ces matériaux peuvent également être présents sous forme stratifiée avec au moins une feuille de métal telle qu'une feuille d'aluminium.

La couche support peut être de toute épaisseur appropriée qui procurera les fonctions de support et de protection souhaitées. De préférence, l'épaisseur de la couche support est comprise entre environ 20 µm et environ 1,5 mm. Avantageusement, la couche support est suffisamment flexible de manière à pouvoir épouser parfaitement le profil de la peau, et à ne pas provoquer chez l'utilisateur, une sensation d'inconfort.

De préférence toutefois, le support est non occlusif. Dans cette dernière hypothèse, on utilise avantageusement un support constitué d'un papier, d'un matériau thermoplastique poreux ou perforé, d'un tissé, d'un non tissé, d'un non tissé perforé.

De préférence, le patch selon l'invention comprend au moins une feuille de protection, pelable avant application du patch.

Lorsque la matrice polymérique est protégée par une couche détachable de protection, celle-ci est enlevée au moment de l'utilisation. Elle peut être constituée en tout matériau imperméable au composé actif ainsi qu'à tout autre composant présent dans la matrice polymérique. Parmi les matériaux pouvant être utilisés, on peut citer de préférence une feuille de papier siliconée ou une feuille de matériau thermoplastique traitée pour la rendre anti-adhérente, par exemple à l'aide d'un vernis. De préférence, cette couche détachable de protection est constituée de polyéthylène. Avantageusement, la feuille de protection est constituée de deux parties se superposant sur une portion médiane du patch, de manière à en faciliter la pose sans que les doigts viennent en contact avec la matrice contenant le ou les actifs. Selon une alternative, la feuille de protection s'étend sur une surface supérieure à la surface du patch, et déborde au delà des limites de ce dernier, de manière à en favoriser le décollement.

De façon connue, les patchs selon la présente invention peuvent être découpés selon un contour approprié correspondant à la zone de surface de peau à traiter, par exemple sous forme de masque pour l'application sur le visage, notamment pour l'application sur le nez, les lèvres, les joues, la zone entre le nez et la lèvre supérieure, sur le front, ou sous toute autre forme nécessaire pour une application sur une zone déterminée du corps, en particulier les cuisses pour un patch à action amincissante ou le buste pour un patch à action raffermissante. En général, la taille d'un patch conforme à l'invention est comprise entre 0,25 cm² et 500 cm², et de préférence, entre 1 cm² et 30 cm².

Le patch peut être conditionné dans une barquette ou dans un sachet de protection formé de deux feuilles d'un complexe papier/film en matière plastique étanche, le papier étant revêtu d'un adhésif scellable à froid, les feuilles étant scellées autour du patch par contact des faces enduites d'adhésif.

De préférence, un tel patch est utilisé pendant une durée d'application relativement courte, comparativement aux patchs conventionnels. Sa durée d'application est de préférence comprise entre 30 s et 15 min, et de préférence encore, comprise entre 30 s et 5 min.

Avantageusement encore, le patch selon l'invention comprend au moins une zone de faible dimension, sous forme d'un onglet, apte à favoriser le décollement du patch.

Une trame constituée d'un tissé ou d'un non tissé, notamment de polyamide, peut être noyée au moins partiellement dans la matrice. La trame peut faire office de support et vice versa. Elle permet de conférer une cohésion plus importante au patch, en particulier lorsque la matrice est du type formant un gel. En outre, dans le cas d'une matrice auto-adhésive, la trame permet de jouer sur l'adhérence du patch à la peau. Avantageusement, une partie desdites particules magnétiques est contenue directement dans la trame, laquelle est ainsi aimantable. Une telle caractéristique peut être avantageuse, notamment en cas d'incompatibilité de l'une des substances actives avec certaines particules magnétiques dans l'hypothèse où elles seraient directement incorporées à la matrice.

Selon un autre aspect de l'invention, on réalise également un procédé de fabrication d'un patch selon la revendication 21

La magnétisation de la matrice polymérique peut être obtenue de différentes manières. A titre d'exemple non limitatif, on utilise des dispositifs d'aimantation du type constitués de bobinages avec ou sans fer doux. De tels bobinages, traversés par un courant impulsionnel crée par un banc d'aimantation, génèrent le champ magnétique nécessaire à l'aimantation de la matrice. Ainsi, on peut utiliser des bancs d'aimantation, tels que figurant au catalogue de la société TE2M® (TECHNIQUES ET MATERIELS MAGNETIQUES) sous les références CE 500, PM 1000 ou PM 2500, qui sont des dispositifs de puissance moyenne à grande, permettant d'aimanter à une cadence élevée des aimants de nuances et de formes diverses.

Avantageusement, le procédé consiste à :
i) déposer la matrice en continu sur un support en bande ;
ii) après polymérisation au moins partielle de la matrice, entraîner ledit support ainsi recouvert de ladite matrice polymérique au travers dudit champ magnétique ;
iii) découper le support magnétisé, sous forme de patchs à la forme et aux dimensions souhaitées ; et
iv) disposer les patchs ainsi découpés dans des sachets de protection.

Alternativement, le procédé peut consister à :
i) déposer la matrice en continu sur un support en bande ;
ii) découper le support recouvert de ladite matrice, sous forme de patchs à la forme et aux dimensions souhaitées;
iii) après polymérisation au moins partielle de la matrice, entraîner les patchs ainsi formés au travers dudit champ magnétique ; et
iv) disposer les patchs ainsi magnétisés dans des sachets de protection.

Alternativement encore, le procédé peut consister à :
i) déposer la matrice en continu sur un support en bande ;
ii) découper le support recouvert de ladite matrice, sous forme de patchs à la forme et aux dimensions souhaitées;
iii) disposer les patchs ainsi découpés dans des sachets de protection ;et
iii) entraîner lesdits sachets au travers dudit champ magnétique.

Alternativement encore, lorsque la matrice polymérique (3) est constituée d'un gel, notamment d'un gel aqueux formé d'au moins un hydrocolloïde, le procédé peut consister à :
i) couler la matrice sous forme liquide, à l'intérieur d'une barquette à la forme et aux dimensions du patch à réaliser, et dans laquelle est disposée éventuellement une trame ajourée, ladite matrice étant coulée dans la barquette via une ouverture formée par un bord de ladite barquette ;
ii) provoquer la polymérisation au moins partielle de la matrice, notamment par réticulation, polyaddition ou polycondensation ; et
iii) soumettre la barquette à un champ magnétique apte à orienter de façon permanente les particules magnétiques de la matrice polymérique.

L'ouverture de la barquette peut ensuite être refermée au moyen d'une pellicule de protection amovible. Alternativement, la barquette peut être introduite ouverte dans un sachet de protection.

Selon un autre aspect de l'invention, le patch peut étre appliqué dans le cadre d'un procédé de traitement de la peau.

Dans la description qui suit, il sera fait référence au dessin dans lequel :
- les figures 1A-1B illustrent de façon schématique un mode de réalisation d'un patch selon l'invention;
- les figures 2A-2B illustrent de façon schématique deux exemples de motifs de magnétisation pouvant être obtenus avec un patch selon l'invention; et
- la figure 3 illustre de façon simplifiée, un mode de réalisation d'un procédé de fabrication d'un patch selon l'invention.
- les figures 4 et 5A-5C illustrent un autre mode de réalisation du patch selon l'invention, ainsi qu'un procédé de fabrication et conditionnement d'un tel patch.

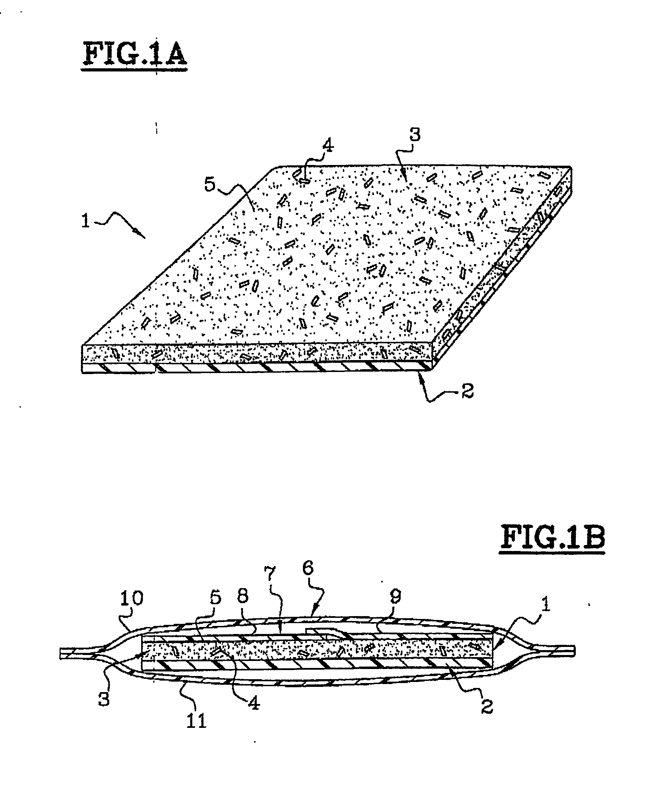

Le patch 1 représenté de façon schématique à la figure 1A comprend un support 2 en polyéthylène et sur lequel est déposé une matrice 3 comportant au moins une substance active, ayant un effet sur la peau, notamment cosmétique, et incorporant des particules magnétiques 4, lesquelles, comme on le verra plus en détail par la suite, sont orientées magnétiquement, de façon permanente. La face 5 de la matrice 3, opposée au support 2, est destinée à être mise en engagement avec la peau, et à y adhérer.

L'adhérence de la face 5 de la matrice 3 sur la peau, peut résulter de l'utilisation d'une matrice auto-adhésive, notamment à base d'un polymère acrylique ou vinylique. Une telle matrice peut être auto-adhésive à sec ou sur peau mouillée. Dans ce dernier cas, de préférence, on incorporera à la matrice des agents hydroabsorbants aptes à assécher localement la peau, et à former des points d'ancrage de la matrice adhésive sur la peau.

Alternativement, l'adhérence sur la peau de face 5 de la matrice 3, peut résulter de la réalisation de la matrice en un gel hydratable, notamment à base de PVP ou de PVA, qui, en séchant, adhère à la peau.

Alternativement encore, l'adhérence sur la peau de la face 5 de la matrice 3, peut résulter d'un effet ventouse résultant de la réalisation de la matrice sous forme d'un gel à forte teneur en eau, comportant au moins un hydrocolloïde, tel que la gomme de gellane.

A la figure 1B, le patch 1 est disposé à l'intérieur d'un sachet de protection 6, la face 5 de la matrice ayant été préalablement recouverte d'une pellicule pelable 7, constituée de deux portions 8 et 9 se chevauchant dans une zone médiane du patch 1. Le sachet de protection 6 est formé de deux feuilles 10, 11 d'un complexe papier/film en matière plastique étanche, le papier étant revêtu d'un adhésif scellable à froid, les feuilles étant scellées autour du patch 1 par contact des faces enduites d'adhésif.

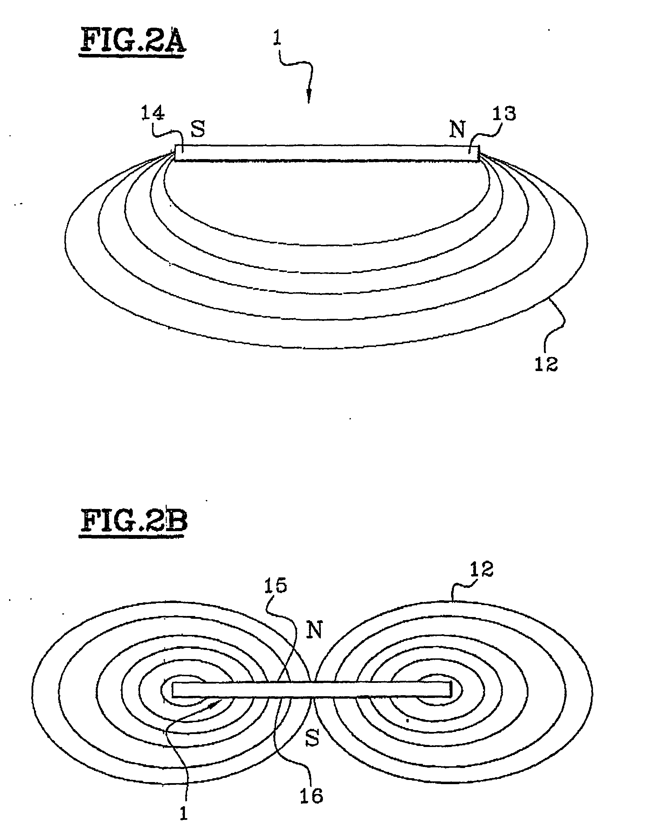

Dans les figures 2A et 2B auxquelles il est maintenant fait référence, ont été illustrées, à titre d'exemple, deux motifs de magnétisation du patch 1. A la figure 2A, le patch est magnétisé de sorte que le "nord" magnétique N soit disposé en une première extrémité 13 du patch 1 tandis que le "sud" magnétique S est disposé en une seconde extrémité 14 du patch 1, opposée à la première. Les lignes de flux 12 s'étendent de la manière représentée schématiquement à la figure 2A.

A la figure 2B, le patch est magnétisé de sorte que le "nord" magnétique S soit disposé sur une première face 15 du patch 1 tandis que le "sud" magnétique est disposé sur une seconde face 16 du patch 1, opposée à la première. Les lignes de flux 12 s'étendent de la manière représentée schématiquement à la figure 2B. Une multitude de motifs différents peuvent être obtenus en fonction notamment de la configuration du (ou des) champ(s) magnétique(s) dans lequel on fait passer le patch, et/ou du nombre de passage du patch 1 dans un tel champ magnétique. Selon une autre alternative, on peut notamment orienter les particules ferro magnétiques de sorte qu'une première polarité soit centrale tandis que l'autre est disposée de manière annulaire tout autour de la première.

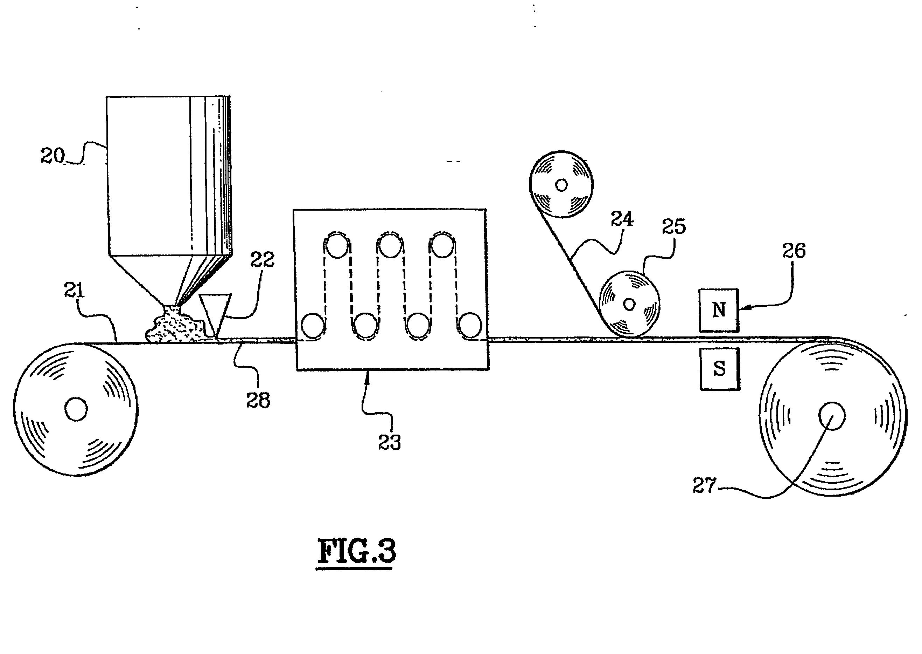

A la figure 3, on a représenté de façon schématique un procédé de fabrication possible d'un patch selon l'invention.

Selon un mode de réalisation préféré de l'invention, on prépare un mélange à partir d'un polymère, notamment un polymère acrylique, solubilisé dans un solvant tel qu'un alcool, par exemple éthylique ou de l'acétate d'éthyle. On ajoute au mélange le ou les actifs, ainsi que les particules ferromagnétiques.

Le mélange est alors introduit dans une trémie 20 et versé sur une feuille de papier siliconé, ou polyéthylène traité 21, constituant la pellicule de protection détachable ou pelable du patch. En aval de la trémie 20 est disposée une racle 22 permettant d'égaliser l'épaisseur de la couche de polymère 28 de la matrice polymérique, cette épaisseur étant généralement comprise entre 10 µm et 300 µm.

On provoque l'évaporation des solvants à une température comprise entre 30 °C et 100 °C, en faisant passer la couche 28 ainsi formée sur la feuille 21, dans un four 23. On obtient ainsi une couche adhésive, sur laquelle on dépose un support 24, notamment un non tissé. L'ensemble est ensuite calandré au moyen d'un rouleau de calandrage 25, et est entraîné en continu au travers d'un banc d'aimantation 26, de manière à orienter de façon permanente les particules ferromagnétiques contenues dans la couche de polymère 28. L'ensemble ainsi formé est alors enroulé sur un noyau 27.

La mise sous forme de patchs individuels s'effectue en faisant passer le support au travers d'un poste de découpe, où il est découpé à la forme et aux dimensions voulues. Préalablement à la découpe, la feuille 21 de papier siliconé ou Polyéthylène traité, et qui à servi à entraîner la couche polymérique 28 au travers des différents postes mentionnés ci-avant du dispositif, peut être remplacée par une structure à deux feuilles se chevauchant dans une zone médiane. Après découpe, les patchs sont ensuite mis en sachets individuels.

Alternativement, l'aimantation des patchs se fait de manière séquentielle, après la mise en sachets de ces derniers.

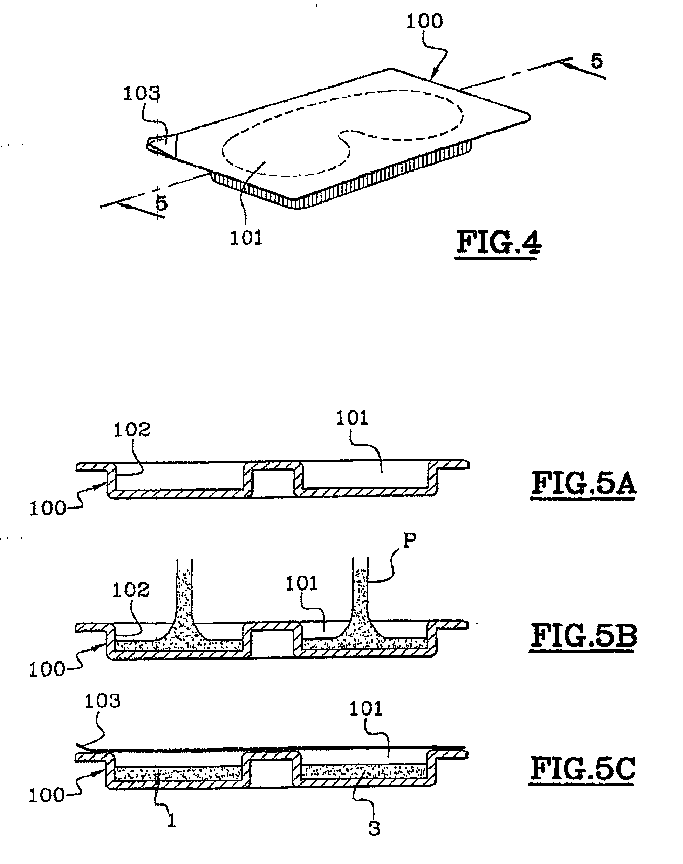

La figure 4 à laquelle il est maintenant fait référence représente une barquette 100 définissant un compartiment 101 à l'intérieur duquel est moulé un patch selon un autre mode de réalisation de l'invention. Selon ce mode de réalisation, la matrice est formée à partir d'un hydrocolloïde, tel qu'une gomme de gellane. De la même manière que pour le mode de réalisation précédent, la matrice comporte des particules ferro magnétiques. La barquette 100 est obtenue par thermoformage ou injection à parois fines d'un matériau tel qu'un polypropylène. Le compartiment 101 est de forme correspondant à celle d'un masque pour les yeux. Ledit compartiment présente un bord délimitant une ouverture, obturée par un opercule thermoscellé 103.

Les figures 5A-5C illustrent une vue en coupe selon la ligne 5-5 de l'ensemble de la figure 4. A la figure 5A, la barquette 100 est posée sur son fond, l'ouverture 102 du compartiment 101 n'étant pas obturée. A la figure 5B, la composition liquide P, apte à former la matrice gélifiée 3 du patch 1, est coulée dans le compartiment 101 via l'ouverture 102. Dans cette phase de coulage, la température de la composition liquide P est de l'ordre de 90 °.

Lors du refroidissement de la composition P, la composition liquide se fige de manière à former un patch gélifié 1. Typiquement, le figeage se produit à une température qui peut être de l'ordre de 60 °C à 70 °C. Avant, pendant, ou après le figeage de la composition, un opercule 103 est thermoscellé de manière à obturer l'ouverture 102. Avant ou après fermeture de la barquette, celle-ci est entraînée au travers d'un banc d'aimantation du type de celui utilisé dans le mode de réalisation précédent, de manière à orienter de façon permanente les particules magnétiques contenues dans la matrice 3.

### EXEMPLE 1

On réalise une composition comprenant en volume :
80,5% de polymère acrylique en solution dans de l'acétate d'éthyle ;
1,5 % d'acide salicylique ;
2 % de caféine ;
3 % de vitamine C ;
8 % de polyacrylate ; et
5 % de particules de ferrite.

Après homogénéisation, le produit est introduit dans une trémie et est étalé à l'aide d'une racle en une couche de 0,8 mm d'épaisseur sur une feuille de polyéthylène d'une épaisseur de 200 µm. Cette feuille peut être préalablement traitée en surface pour réduire son adhérence. L'ensemble est chauffé à 60° C de manière à permettre l'évaporation du solvant. On applique ensuite sur la surface libre de la couche polymérique, un non tissé, formé à 30 % de Polyéthylène téréphtalate, et à 70 % de viscose, lequel non tissé constitue la couche support du patch, et on procède au calandrage de l'ensemble. On obtient ainsi un ensemble comportant une couche support et une couche de polymère auto-adhésive formée d'une matrice polyacrylique, cet ensemble comprenant en outre, une couche détachable de protection. L'ensemble ainsi formé est alors entraîné dans un banc d'aimantation commercialisé sous la référence PM 1000 par la société TE2M® , puis découpé à la forme et aux dimensions souhaitées.

Les patchs après découpe sont ensuite conditionnés dans des sachets de polyéthylène.

Un tel patch est particulièrement efficace pour le traitement des rides et poches aux contours des yeux, la pénétration des actifs du patch étant favorisée par la présence des particules de ferrite magnétisées de façon permanente, et qui ont une action sur la micro circulation au niveau de la zone d'application du patch.

### EXEMPLE II

On réalise une composition comprenant en volume :
77,5% de polymère acrylique en solution dans de l'acétate d'éthyle ;
3 % de Glycérine ;
10 % de ferrite ;
5 % de fibres de coton ;
0,5 % d'acide Kojique ;
3 % de caféine ; et
1 % de thé vert.

Le mode opératoire correspond sensiblement à celui décrit dans l'exemple précédent.

Un tel patch a un effet relaxant, éclaircissant et défatiguant, l'action des actifs étant renforcée par la présence des particules de ferrite, magnétisées de façon permanente, et qui contribuent à rendre la peau plus réceptive à de tels actifs.

## Revendications

1. Patch (1) comprenant au moins une matrice polymérique (3) et un support (2) sur lequel est déposée ladite matrice polymérique, tel qu'une surface (5) de ladite matrice polymérique, adhésive ou apte à le devenir, notamment après hydratation, est destinée à être mise en engagement avec la peau, ladite matrice (3) contenant au moins une substance active apte à avoir une action, notamment cosmétique sur la peau, et des particules magnétiques (4), en dispersion dans la matrice (3), lesdites particules étant, sur au moins une portion de la surface du patch (1), orientées de façon permanente.

2. Patch selon la revendication 1 **caractérisé en ce que** lesdites particules magnétiques (4) sont notamment des ferrites.

3. Patch selon la revendication 1 ou 2 **caractérisé en ce que** le pourcentage en volume desdites particules magnétiques (4) à l'intérieur de la matrice polymérique va de 0,2 à 80 %, et de préférence, de 1 % à 30 %, et de préférence encore, de 2 % à 15 %.

4. Patch selon l'une quelconque des revendications 1 à 3 **caractérisé en ce que** lesdites particules (4) sont enrobées, notamment de polyuréthanne, d'epoxy, de polyester, de polyamide, ou de cyanoacrylate.

5. Patch selon l'une quelconque des revendications 1 à 4 **caractérisé en ce que** les particules magnétiques (4) sont orientées de sorte que, sur au moins une portion de la surface du patch (1), les lignes de champ magnétique (12) générées, s'étendent depuis une première face (15) du patch jusqu'à une seconde face (16), opposée à la première.

6. Patch selon l'une quelconque des revendications 1 à 5 **caractérisé en ce que** les particules magnétiques (4) sont orientées de sorte que, sur au moins une portion de la surface du patch (1), les lignes de champ magnétique (12) générées s'étendent depuis un premier bord (13) du patch jusqu'à un second bord (14), opposé au premier.

7. Patch selon l'une quelconque des revendications 1 à 6 **caractérisé en ce que** la (ou les) substances active(s) est (sont) choisi(es) parmi les agents amincissants, nettoyants, anti-oxydants, anti-radicaux libres, hydratants, dépigmentants, liporégulateurs, anti-acnéiques, anti-séborrhéiques, anti-vieillissement, adoucissants, anti-rides, kératolitiques, anti-inflammatoires, rafraîchissants, cicatrisants, protecteurs vasculaires, anti-bactériens, antifongiques, anti-perspirants, déodorants, conditionneurs de la peau, insensibilisants, immunomodulateurs et nourrissants, absorbeurs d'humidité (coton, polyacrylate), de sébum (Orgasol).

8. Patch selon l'une quelconque des revendications 1 à 7 **caractérisé en ce que** la matrice polymérique (3) comporte au moins une substance active hydrosoluble choisie parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

9. Patch selon l'une quelconque des revendications 1 à 8 **caractérisé en ce que** la matrice polymérique (3) comporte au moins une substance active liposoluble choisie parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'a-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

10. Patch selon l'une quelconque des revendications 1 à 9 **caractérisé en ce que** la matrice polymérique (3) contient au moins une substance active ayant un effet nettoyant sur la peau, ledit patch comportant une couche colorée de manière à pouvoir, de visu, quantifier et/ou qualifier les impuretés prélevées de la peau par ladite surface adhésive (5).

11. Patch selon la revendication 10 **caractérisé en ce que** la matrice polymérique (3) contient au moins une substance active choisie parmi les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide tartrique, l'acide malique, l'acide citrique, l'acide mandélique et de manière générale les acides de fruits et les bêta-hydroxyacides comme l'acide salicylique et ses dérivés notamment alcoylés comme, l'acide n-octanoyl-5-salicylique ; de la poudre de Kaolin, des particules de polyamide (ORGASOL® ), des cires, du miel, de la terre de Sienne, des tanins ou des sels minéraux.

12. Patch selon l'une quelconque des revendications 1 à 11 **caractérisé en ce que** la matrice polymérique (3) est auto-adhésive, et formée notamment à base d'un polymère acrylique, vinylique, d'un silicone, d'un polyuréthanne, d'un latex ou d'un butyle.

13. Patch selon la revendication 12 **caractérisé en ce que** la matrice polymérique (3) contient au moins un agent hydroabsorbant, choisi parmi les polyacrylates réticulés superabsorbants, l'alcool polyvinylique, les polymères carboxyvinyliques, les dérivés semi-synthétiques de la cellulose, les amidons, les gommes de guar, arabique ou adragante, la caséine, les phytocolloïdes, les fibres de coton et la gélatine.

14. Patch selon l'une quelconque des revendications 1 à 11 **caractérisé en ce que** la matrice polymérique (3) est formée d'au moins un polymère hydrosoluble, notamment un PVP ou PVA, apte, après hydratation, à former un gel qui, en séchant adhère à la peau.

15. Patch selon l'une quelconque des revendications 1 à 11 **caractérisé en ce que** la matrice polymérique (3) est formée d'un gel aqueux à base d'au moins un hydrocolloïde, tel que la gomme de gellane ou la gomme de xanthane.

16. Patch selon l'une quelconque des revendications 1 à 15, **caractérisé en ce que** le support (2) est un support occlusif constitué notamment d'un matériau thermoplastique, choisi parmi les polyéthylènes haute et basse densité, les polypropylènes, les polychlorure de vinyle, les copolymères d'éthylène et d'acétate de vinyle, les polyesters et les polyuréthannes, ou d'un complexe de tels matériaux, ou un support non occlusif constitué notamment d'un papier, d'un matériau thermoplastique poreux ou perforé, d'un tissé, d'un non tissé, d'un non tissé perforé.

17. Patch selon l'une quelconque des revendications 1 à 16 **caractérisé en ce qu'**il comprend au moins une feuille de protection (7), notamment sous forme de deux parties (8, 9) se superposant sur une portion médiane du patch, pelable avant application du patch.

18. Patch selon l'une quelconque des revendications précédentes **caractérisé en qu'**une trame constituée d'un tissé ou d'un non tissé, notamment de polyamide, est noyée au moins partiellement dans la matrice polymérique (3).

19. Patch selon la revendication 18 **caractérisé en ce que** une partie au moins desdites particules magnétiques (4) est contenue dans ladite trame.

20. Patch selon l'une quelconque des revendications précédentes, **caractérisé par le fait qu'**il est conditionné dans un sachet de protection (6) formé de deux feuilles (10, 11) d'un complexe papier/film en matière plastique étanche, le papier étant revêtu d'un adhésif scellable à froid, les feuilles étant scellées autour du patch par contact des faces enduites d'adhésif.

21. Procédé de fabrication d'un patch (1) consistant à :
a) former une matrice polymérique (3) et la déposer sur un support (2), tel qu'une surface (5) de cette matrice polymérique, adhésive ou apte à le devenir, notamment après hydratation, est destinée à être mise en engagement avec la peau, ladite matrice (3) contenant au moins une substance active apte à avoir une action, notamment cosmétique sur la peau, et des particules magnétiques (4), en dispersion dans la matrice ; et
b) après polymérisation au moins partielle de la matrice, faire passer ladite matrice polymérique (3) dans un champ magnétique (26) de manière à, sur une partie au moins de la surface du patch, orienter de manière permanente lesdites particules (4).

22. Procédé selon la revendication 21 **caractérisé en ce qu'**il consiste à :
i) déposer la matrice (3) en continu sur un support en bande (24) ;
ii) après polymérisation au moins partielle de la matrice (3), entraîner ledit support ainsi recouvert de ladite matrice polymérique (3) au travers dudit champ magnétique (26) ;
iii) découper le support magnétisé, sous forme de patchs (1) à la forme et aux dimensions souhaitées; et
iv) disposer les patchs ainsi découpés dans des sachets de protection (6).

23. Procédé selon la revendication 21 **caractérisé en ce qu'**il consiste à :
i) déposer la matrice (3) en continu sur un support en bande (24) ;
ii) découper le support recouvert de ladite matrice polymérique (3), sous forme de patchs (1) à la forme et aux dimensions souhaitées;
iii) entraîner les patchs ainsi formés au travers dudit champ magnétique (26) ; et
iv) disposer les patchs ainsi magnétisés dans des sachets de protection (6).

24. Procédé selon la revendication 21 **caractérisé en ce qu'**il consiste à :
i) déposer la matrice (3) en continu sur un support en bande (24 ;
ii) découper le support recouvert de ladite matrice, sous forme de patchs (1) à la forme et aux dimensions souhaitées ;
iii) disposer les patchs ainsi découpés dans des sachets de protection (6) ;et
iv) entraîner lesdits sachets au travers dudit champ magnétique (26).

25. Procédé selon la revendication 21 **caractérisé en ce que** la matrice polymérique (3) est constituée d'un gel, notamment d'un gel aqueux formé d'au moins un hydrocolloïde, ledit procédé consistant à :
i) couler la matrice sous forme liquide, à l'intérieur d'une barquette (100) à la forme et aux dimensions du patch à réaliser, et dans laquelle est disposée éventuellement une trame ajourée, ladite matrice (3) étant coulée dans la barquette via une ouverture (102) formée par un bord de ladite barquette ;
ii) provoquer la solidification de la matrice (3) par polymérisation au moins partielle ; et
iii) soumettre la barquette (100) à un champ magnétique apte à orienter de façon permanente les particules magnétiques de la matrice (3).

26. Procédé selon la revendication 25 **caractérisé en ce qu'**il comprend en outre une étape consistant à :
iv) refermer l'ouverture de la barquette au moyen d'une pellicule de protection amovible (103).

27. Procédé selon la revendication 25 **caractérisé en ce que**, avant ou après l'étape iii), la barquette (100) est introduite dans un sachet de protection.

28. Procédé de traitement non-thérapeutique de la peau **caractérisé en ce qu'**on applique un patch selon l'une quelconque des revendications 1 à 20 sur la peau.

## Claims

1. Patch (1) comprising at least one polymer matrix (3) and a support (2) onto which is deposited the said polymer matrix, such that one surface (5) of the said polymer matrix, which is adhesive or capable of becoming adhesive, in particular after moistening, is intended to be placed in contact with the skin, the said matrix (3) containing at least one active substance capable of having an action, in particular a cosmetic action, on the skin, and magnetic particles (4) dispersed in the matrix (3), the said particles being permanently aligned over at least a portion of the area of the patch (1).

2. Patch according to Claim 1, **characterized in that** the said magnetic particles (4) are in particular ferrites.

3. Patch according to Claim 1 or 2, **characterized in that** the percentage by volume of the said magnetic particles (4) in the polymer matrix ranges from 0.2 to 80%, preferably from 1% to 30% and even more preferably from 2% to 15%.

4. Patch according to any one of Claims 1 to 3, **characterized in that** the said particles (4) are coated, in particular with polyurethane, epoxy resin, polyester, polyamide or cyanoacrylate.

5. Patch according to any one of Claims 1 to 4, **characterized in that** the magnetic particles (4) are aligned such that the magnetic field lines (12) generated extend, over at least a portion of the area of the patch (1), from a first face (15) of the patch to a second face (16), opposite the first face.

6. Patch according to any one of Claims 1 to 5, **characterized in that** the magnetic particles (4) are aligned such that the magnetic field lines (12) generated extend, over at least a portion of the area of the patch (1), from a first edge (13) of the patch to a second edge (14), opposite the first edge.

7. Patch according to any one of Claims 1 to 6, **characterized in that** the active substance(s) is(are) chosen from weight-reducing agents, cleansing agents, antioxidants, free-radical scavengers, moisturizers, depigmenting agents, liporegulators, anti-acne agents, anti-seborrhoeic agents, anti-ageing agents, softeners, anti-wrinkle agents, keratolytic agents, anti-inflammatory agents, refreshing agents, cicatrizing agents, vascular protectors, antibacterial agents, antifungal agents, antiperspirants, deodorants, skin conditioners, desensitizers, immunomodulators and nourishing agents, moisture absorbers (cotton, polyacrylate) and sebum absorbers (Orgasol).

8. Patch according to any one of Claims 1 to 7, **characterized in that** the polymer matrix (3) comprises at least one water-soluble active substance chosen from the following compounds: ascorbic acid and its biologically compatible salts, enzymes, antibiotics, components with a tensioning effect, α-hydroxy acids and their salts, polyhydroxy acids, sugars and sugar derivatives, urea, amino acids, oligopeptides, water-soluble plant extracts, yeasts, protein hydrolysates, hyaluronic acid, mucopolysaccharides, vitamins B₂, B₆, H and PP, panthenol, folic acid, acetylsalicylic acid, allantoin, glycyrrhetic acid, kojic acid and hydroquinone.

9. Patch according to any one of Claims 1 to 8, **characterized in that** the polymer matrix (3) comprises at least one liposoluble active substance chosen from the following compounds: D-α-tocopherol, DL-α-tocopherol, D-α-tocopheryl acetate, DL-α-tocopheryl acetate, ascorbyl palmitate, vitamin F and vitamin F glycerides, D vitamins, vitamin D₂, vitamin D₃, retinol, retinol esters, retinyl palmitate, retinyl propionate, β-carotene, D-panthenol, farnesol, farnesyl acetate; jojoba oil and blackcurrant oil rich in essential fatty acids; keratolytic agents such as salicylic acid, its salts and its esters, 5-n-octanoylsalicylic acid and its esters, alkyl esters of α-hydroxy acids such as citric acid, lactic acid or glycolic acid; asiatic acid, madecassic acid, asiaticoside, total extract of Centella asiatica, β-glycyrrhetinic acid, α-bisabolol, ceramides such as 2-oleoylamino-1,3-octadecane; phytanetriol, milk sphingomyelin, phospholipids of marine origin which are rich in polyunsaturated essential fatty acids, ethoxyquine; extract of rosemary, extract of balm, quercetin, extract of dried microalgae, steroidal anti-inflammatory agents.

10. Patch according to any one of Claims 1 to 9, **characterized in that** the polymer matrix (3) contains at least one active substance which has a cleansing effect on the skin, the said patch comprising a layer which is coloured so as to be able visually to quantify and/or qualify the impurities taken up from the skin by the said adhesive surface (5).

11. Patch according to Claim 10, **characterized in that** the polymer matrix (3) contains at least one active substance chosen from keratolytic agents such as α- and β-hydroxycarboxylic or β-keto carboxylic acids, their salts, amides or esters and more particularly α-hydroxy acids such as glycolic acid, lactic acid, tartaric acid, malic acid, citric acid, mandelic acid and fruit acids in general, and β-hydroxy acids such as salicylic acid and its derivatives, in particular its alkyl derivatives, such as 5-n-octanoylsalicylic acid; Kaolin powder, polyamide (Orgasol® ) particles, waxes, honey, Sienna, tannins or mineral salts.

12. Patch according to any one of Claims 1 to 11, **characterized in that** the polymer matrix (3) is self-adhesive and is based in particular on an acrylic or vinyl polymer, a silicone, a polyurethane, a latex or a butyl.

13. Patch according to Claim 12, **characterized in that** the polymer matrix (3) contains at least one water-absorbing agent chosen from superabsorbent crosslinked polyacrylates, polyvinyl alcohol, carboxyvinyl polymers, semisynthetic cellulose derivatives, starches, guar gums, gum arabic or gum tragacanth, casein, phytocolloids, cotton fibres and gelatin.

14. Patch according to any one of Claims 1 to 11, **characterized in that** the polymer matrix (3) is formed from at least one water-soluble polymer, in particular a PVP or PVA, which is capable, after moistening, of forming a gel which, on drying, adheres to the skin.

15. Patch according to any one of Claims 1 to 11, **characterized in that** the polymer matrix (3) is formed from an aqueous gel based on at least one hydrocolloid, such as gellan gum or xanthan gum.

16. Patch according to any one of Claims 1 to 15, **characterized in that** the support (2) is an occlusive support consisting in particular of a thermoplastic material chosen from high-density and low-density polyethylenes, polypropylenes, polyvinyl chlorides, copolymers of ethylene and of vinyl acetate, polyesters and polyurethanes, or a complex of such materials, or a non-occlusive support consisting in particular of a paper, a porous or perforated thermoplastic material, a woven, a nonwoven or a perforated nonwoven.

17. Patch according to any one of Claims 1 to 16, **characterized in that** it comprises at least one protective sheet (7), in particular in the form of two parts (8, 9) which superimpose over a mid-portion of the patch, which can be peeled off before the patch is applied.

18. Patch according to any one of the preceding claims, **characterized in that** a frame made of a woven or a nonwoven, in particular of polyamide, is at least partially immersed in the polymer matrix (3).

19. Patch according to Claim 18, **characterized in that** at least some of the said magnetic particles (4) are contained in the said frame.

20. Patch according to any one of the preceding claims, **characterized in that** it is packaged in a protective sachet (6) formed from two sheets (10, 11) of a paper/film complex made of leakproof plastic, the paper being coated with a cold-sealable adhesive, the sheets being sealed around the patch by contact with the faces coated with adhesive.

21. Process for manufacturing a patch (1), which consists in:
a)forming a polymer matrix (3) and placing it on a support (2) such that one surface (5) of this polymer matrix, which is adhesive or capable of becoming adhesive, in particular after moistening, is intended to be placed in contact with the skin, the said matrix (3) containing at least one active substance capable of having an action, in particular a cosmetic action, on the skin, and magnetic particles (4) dispersed in the matrix; and
b)after at least partial polymerization of the matrix, in placing the said polymer matrix (3) in a magnetic field (26) so as to permanently align the said particles (4), over at least a portion of the area of the patch.

22. Process according to Claim 21, **characterized in that** it consists in:
i)depositing the matrix (3) continuously onto a strip of support (24);
ii)after at least partial polymerization of the matrix (3), conveying the said support thus coated with the said polymer matrix (3) through the said magnetic field (26);
iii)cutting up the magnetized support into the form of patches (1) of the desired shape and size; and
iv)placing the patches thus cut up in protective sachets (6).

23. Process according to Claim 21, **characterized in that** it consists in:
i)depositing the matrix (3) continuously on a strip of support (24);
ii)cutting up the support coated with the said polymer matrix (3) into the form of patches (1) of the desired shape and size;
iii)conveying the patches thus formed through the said magnetic field (26); and
iv)placing the patches thus magnetized in protective sachets (6).

24. Process according to Claim 21, **characterized in that** it consists in:
i)depositing the matrix (3) continuously onto a strip of support (24);
ii)cutting up the support coated with the said matrix into the form of patches (1) of the desired shape and size;
iii)placing the patches thus cut up in protective sachets (6); and
iv)conveying the said sachets through the said magnetic field (26).

25. Process according to Claim 21, **characterized in that** the polymer matrix (3) consists of a gel, in particular an aqueous gel formed from at least one hydrocolloid, the said process consisting in:
i)pouring the matrix in liquid form into a tray (100) of the shape and size of the patch to be made, and in which is optionally placed an open-worked frame, the said matrix (3) being poured into the tray via an aperture (102) formed by one edge of the said tray;
ii)bringing about the solidification of the matrix (3) by at least partial polymerization; and
iii)subjecting the tray (100) to a magnetic field capable of permanently aligning the magnetic particles of the matrix (3).

26. Process according to Claim 25, **characterized in that** it also comprises a step which consists in:
iv)closing the aperture of the tray with a removable protective foil (103).

27. Process according to Claim 25, **characterized in that**, before or after step iii), the tray (100) is introduced into a protective sachet.

28. Non-therapeutic skin treatment process, **characterized in that** a patch according to any one of Claims 1 to 20 is applied to the skin.

## Patentansprüche

1. Pflaster (1), das mindestens eine Polymermatrix (3) und einen Träger (2) umfasst, auf den die Polymermatrix aufgebracht wurde, wobei eine Oberfläche (5) der Polymermatrix, die adhäsiv ist oder insbesondere nach Hydratisierung adhäsiv werden kann, dazu vorgesehen ist, mit der Haut in Kontakt zu kommen, wobei die Matrix (3) mindestens einen Wirkstoff, der auf die Haut eine Wirkung und insbesondere eine kosmetische Wirkung haben kann, und dispergiert in der Matrix (3) magnetische Partikel (4) enthält, wobei die Partikel zumindest an einem Teil der Oberfläche des Pflasters (1) in permanenter Weise orientiert sind.

2. Pflaster nach Anspruch 1, **dadurch gekennzeichnet, dass** die magnetischen Partikel (4) insbesondere Ferrite sind.

3. Pflaster nach Anspruch 1 oder 2, **dadurch gekennzeichnet, dass** der prozentuale Volumenanteil der magnetischen Partikel (4) im Inneren der Polymermatrix im Bereich von 0,2 bis 80 %, vorzugsweise 1 bis 30 % und noch bevorzugter 2 bis 15 % liegt.

4. Pflaster nach einem der Ansprüche 1 bis 3, **dadurch gekennzeichnet, dass** die Partikel (4) insbesondere mit Polyurethan, Epoxy, Polyester, Polyamid oder Cyanoacrylat umhüllt sind.

5. Pflaster nach einem der Ansprüche 1 bis 4, **dadurch gekennzeichnet, dass** die magnetischen Partikel (4) so orientiert sind, dass sich an zumindest einem Teil der Oberfläche des Pflasters (1) die erzeugten Magnetfeldlinien (12) von einer ersten Fläche (15) des Pflasters bis zu einer zweiten Fläche (16) erstrecken, die der ersten Fläche gegenüberliegt.

6. Pflaster nach einem der Ansprüche 1 bis 5, **dadurch gekennzeichnet, dass** die magnetischen Partikel (4) so orientiert sind, dass sich an zumindest einem Teil der Oberfläche des Pflasters (1) die erzeugten Magnetfeldlinien (12) von einem ersten Rand (13) des Pflasters bis zu einem zweiten Rand (14) erstrecken, der dem ersten gegenüberliegt.

7. Pflaster nach einem der Ansprüche 1 bis 6, **dadurch gekennzeichnet, dass** der oder die Wirkstoff(e) unter den schlanker machenden Wirkstoffen, reinigenden Wirkstoffen, Antioxidantien, Radikalfängern für freie Radikale, Hydratisierungsmitteln, Depigmentierungsmitteln, Liporegulatoren, Wirkstoffen gegen Akne, Wirkstoffen gegen Seborrhoe, Wirkstoffen gegen Alterung, reizlindernden Wirkstoffen, Wirkstoffen gegen Falten, Keratolytika, entzündungshemmenden Wirkstoffen, erfrischenden Mitteln, Vernarbungsmitteln, vaskulären Schutzmitteln, antibakteriellen Wirkstoffen, Fungiziden, Antiperspirantien, Deodorants, Hautkonditionierern, unempfindlich machenden Wirkstoffen, Immunomodulatoren und Nährstoffen, Feuchtigkeitsabsorbern (Baumwolle, Polyacrylat) und Sebumabsorbern (Orgasol) ausgewählt sind.

8. Pflaster nach einem der Ansprüche 1 bis 7, **dadurch gekennzeichnet, dass** die Polymermatrix (3) mindestens einen wasserlöslichen Wirkstoff enthält, der unter den folgenden Verbindungen ausgewählt ist: Ascorbinsäure und ihren biologisch kompatiblen Salzen, Enzymen, Antibiotika, Komponenten mit straffender Wirkung, α-Hydroxysäuren und ihren Salzen, hydroxylierten Polysäuren, Zuckern und deren Derivaten, Harnstoff, Aminosäuren, Oligopeptiden, wasserlöslichen pflanzlichen Extrakten und Hefeextrakten, Proteinhydrolysaten, Hyluronsäure, Mucopolysacchariden, Vitamin B₂, B₆, H, PP, Panthenol, Folsäure, Acetylsalicylsäure, Allantoin, Glycyrrhetinsäure, Kojisäure und Hydrochinon.

9. Pflaster nach einem der Ansprüche 1 bis 8, **dadurch gekennzeichnet, dass** die Polymermatrix (3) mindestens einen fettlöslichen Wirkstoff enthält, der unter den folgenden Verbindungen ausgewählt ist: D-α-Tocopherol, DL-α-Tocopherol, D-α-Tocopherolacetat, DL-α-Tocopherolacetat, Ascorbylpalmitat, Vitamin F und Glyceride von Vitamin F, D-Vitamine, Vitamin D₂, Vitamin D₃, Retinol, Retinolester, Retinolpalmitat, Retinolpropionat, β-Carotin, D-Panthenol, Farnesol, Farnesylacetat; Jojobaöl und Johannisbeeröl mit einem hohen Anteil an essentiellen Fettsäuren; Keratolytika, wie Salicylsäure und ihre Salze und ihre Ester, 5-n-Octanoylsalicylsäure und ihren Estern, Alkylestern von α-Hydroxysäuren, wie Citronensäure, Milchsäure, Glykolsäure; Asiatsäure, Madecassäure, Asiaticosid, dem Gesamtextrakt von Centella asiatica, β-Glycyrrhetinsäure, α-Bisabolol, Ceramide, wie 1,3-Oleylamino-octadecan; Phytantriol, Milchsphingomyclin, Phospholipiden mariner Herkunft mit einem hohen Anteil an mehrfach ungesättigten essentiellen Fettsäuren, Ethoxyquin; Rosmarinextrakt, Melissenextrakt, Quercetin, Extrakten aus getrockneten Mikroalgen, steroidalen entzündungshemmenden Wirkstoffen.

10. Pflaster nach einem der Ansprüche 1 bis 9, **dadurch gekennzeichnet, dass** die Polymermatrix (3) mindestens einen Wirkstoff enthält, der auf die Haut reinigend wirkt, wobei das Pflaster eine farbige Schicht aufweist, damit visuell die durch die adhäsive Oberfläche (3) abgenommenen Unreinheiten der Haut quantifiziert und/oder qualifiziert werden können.

11. Pflaster nach Anspruch 10, **dadurch gekennzeichnet, dass** die Polymermatrix (3) mindestens einen Wirkstoff enthält, der unter den Keratolytika, wie α- und β-Hydroxycarbonsäuren oder β-Ketocarbonsäuren, ihren Salzen, Amiden oder Estern und insbesondere α-Hydroxysäuren, wie Glykolsäure, Milchsäure, Weinsäure, Äpfelsäure, Citronensäure, Mandelsäure und allgemein Fruchtsäuren und β-Hydroxysäuren, wie Salicylsäure und ihren Derivaten und insbesondere den alkylierten Derivaten, wie 5-n-Octanoylsalicylsäure; Kaolinpulver, Polyamidpartikeln (ORGASOL® ), Wachsen, Honig, Terra de Siena, Tanninen oder anorganischen Salzen ausgewählt ist.

12. Pflaster nach einem der Ansprüche 1 bis 11, **dadurch gekennzeichnet, dass** die Polymermatrix (3) selbsthaftend ist und insbesondere auf der Basis eines Acrylpolymers, Vinylpolymers, Silicons, Polyurethans, Latex oder einer Butylverbindung gebildet ist.

13. Pflaster nach Anspruch 12, **dadurch gekennzeichnet, dass** die Polymermatrix (3) mindestens einen wasserabsorbierenden Stoff enthält, der insbesondere unter den superabsorbierenden vernetzten Polyacrylaten, Polyvinylalkohol, Carboxyvinylpolymeren, halbsynthetischen Cellulosederivaten, Stärkeverbindungen, Guargummi, Gummi arabicum, Tragant, Casein, Phytocolloiden, Baumwollfasern und Gelatine ausgewählt ist.

14. Pflaster nach einem der Ansprüche 1 bis 11, **dadurch gekennzeichnet, dass** die Polymermatrix (3) aus mindestens einem wasserlöslichen Polymer, insbesondere PVP oder PVA, gebildet ist, das nach Hydratisierung ein Gel bilden kann, das beim Trocknen an der Haut haftet.

15. Pflaster nach einem der Ansprüche 1 bis 11, **dadurch gekennzeichnet, dass** die Polymermatrix (3) aus einem wässrigen Gel auf der Basis mindestens eines Hydrokolloids gebildet ist, wie Gellangummi oder Xanthangummi.

16. Pflaster nach einem der Ansprüche 1 bis 15, **dadurch gekennzeichnet, dass** der Träger (2) ein okklusiver Träger, der insbesondere aus einem thermoplastischen Material, das unter den Polyethylenen mit hoher oder niedriger Dichte, Polypropylenen, Polyvinylchloriden, Copolymeren von Ethylen und Vinylacetat, Polyestern und Polyurethanen ausgewählt ist, oder einem Komplex dieser Materialien besteht, oder ein nicht okklusiver Träger ist, der insbesondere aus einem Papier, einem porösen oder perforierten thermoplastischen Material, einem Gewebe, einem Nonwoven oder einem perforierten Nonwoven.

17. Pflaster nach einem der Ansprüche 1 bis 16, **dadurch gekennzeichnet, dass** es mindestens eine Schutzfolie (7) umfasst, insbesondere in Form von zwei Teilen (8, 9), die sich in einem mittleren Bereich des Pflasters überlappen und vor dem Aufbringen des Pflasters abgezogen werden können.

18. Pflaster nach einem der vorhergehenden Ansprüche, **dadurch gekennzeichnet, dass** ein Geflecht, das aus einem Gewebe oder einem Nonwoven besteht, insbesondere aus Polyamid, zumindest teilweise in die Polymermatrix (3) eingebettet ist.

19. Pflaster nach Anspruch 18, **dadurch gekennzeichnet, dass** zumindest ein Teil der Magnetpartikel (4) in dem Geflecht enthalten ist.

20. Pflaster nach einem der vorhergehenden Ansprüche, **dadurch gekennzeichnet, dass** es in einem Schutzbeutel (6) konfektioniert ist, der aus zwei Folien (10, 11) eines dichten Papier/Kunststofffolie-Komplexes besteht, wobei das Papier mit einem in der Kälte adhäsiven Klebstoff beschichtet ist, wobei die Folien durch Kontakt der mit dem Klebstoff beschichteten Seiten um das Pflaster geklebt sind.

21. Verfahren zur Herstellung eines Pflasters (1), das darin besteht:
a) eine Polymermatrix (3) zu formen und auf einem Träger (2) aufzubringen, wobei eine Oberfläche (5) der Polymermatrix, die adhäsiv ist oder insbesondere nach Hydratisierung adhäsiv werden kann, mit der Haut in Kontakt kommen soll, wobei die Matrix (3) mindestens einen Wirkstoff, der befähigt ist, eine Wirkung und insbesondere eine kosmetische Wirkung auf die Haut auszuüben, und dispergiert in der Matrix magnetische Partikel (4) enthält; und
b) nach der zumindest teilweisen Polymerisation der Matrix die Polymermatrix (3) so durch ein Magnetfeld (26) zu führen, dass zumindest an einem Teil der Oberfläche des Pflasters die Partikel (4) permanent ausgerichtet werden.

22. Verfahren nach Anspruch 21, **dadurch gekennzeichnet, dass** es darin besteht:
i) die Matrix (3) kontinuierlich auf einen bandförmigen Träger (24) aufzubringen;
ii) nach der zumindest teilweisen Polymerisation der Matrix (3) den so mit der Polymermatrix (3) bedeckten Träger durch das Magnetfeld (26) zu führen;
iii) den magnetisierten Träger in Form von Pflastern (1) in der gewünschten Form und der gewünschten Abmessung zu schneiden; und
iv) die derart geschnitten Pflaster in Schutzhüllen (6) zu geben.

23. Verfahren nach Anspruch 21, **dadurch gekennzeichnet, dass** es darin besteht:
i) die Matrix (3) kontinuierlich auf einen bandförmigen Träger (24) aufzubringen;
ii) den mit der Polymermatrix bedeckten Träger in Form von Pflastern (1) in der gewünschten Form und den gewünschten Abmessungen zu schneiden;
iii) die so geformten Pflaster durch ein Magnetfeld (26) zu führen; und
iv) die derart magnetisierten Pflaster in Schutzhüllen (6) anzubringen.

24. Verfahren nach Anspruch 21, **dadurch gekennzeichnet, dass** es darin besteht:
i) die Matrix (3) kontinuierlich auf einen bandförmigen Träger (24) aufzubringen;
ii) den mit der Matrix bedeckten Träger in Form von Pflastern (1) in der gewünschten Form und den gewünschten Abmessungen zu schneiden;
iii) die derart geschnittenen Pflaster in Schutzhüllen (6) zu geben; und
iv) die Schutzhüllen durch das Magnetfeld (26) zu führen.

25. Verfahren nach Anspruch 21, **dadurch gekennzeichnet, dass** die Polymermatrix (3) aus einem Gel und insbesondere aus einem wässrigen Gel besteht, das aus mindestens einem Hydrokolloid gebildet ist, wobei das Verfahren darin besteht:
i) die Matrix in flüssiger Form ins Innere einer Form (100) in der Form und in den Abmessungen des herzustellenden Pflasters zu gießen, in der gegebenenfalls ein Geflecht angeordnet ist, wobei die Matrix (3) über eine Öffnung (102) in die Form gegossen wird, die an einem Rand der Form ausgebildet ist;
ii) die Verfestigung der Matrix (3) durch zumindest teilweise Polymerisation hervorzurufen; und
iii) die Form (100) einem Magnetfeld auszusetzen, das geeignet ist, die magnetischen Partikel der Matrix (3) permanent auszurichten.

26. Verfahren nach Anspruch 25, **dadurch gekennzeichnet, dass** es ferner einen Schritt umfasst, der darin besteht:
iv) die Öffnung der Form mit mindestens einer ablösbaren Schutzschicht (103) zu verschließen.

27. Verfahren nach Anspruch 25, **dadurch gekennzeichnet, dass** vor oder nach Schritt iii) die Form (100) in eine Schutzhülle gegeben wird.

28. Verfahren zur nicht-therapeutischen Behandlung der Haut, **dadurch gekennzeichnet, dass** ein Pflaster nach einem der Ansprüche 1 bis 20 auf die Haut aufgebracht wird.
